# EUROPEAN PATENT APPLICATION

(11) **EP 1 892 521 A1**
(43) Date of publication of application: **27.02.2008**
(21) Application number: 06757028.3
(22) Date of filing: 06.06.2006
(51) Int. Cl.: G01N 21/35

(54) **BIOMETRIC INFORMATION MEASURING SENSOR**

(30) Priority: 07.06.2005 JP 2005166888
(71) Applicant: Omron Healthcare Co., Ltd., Kyoto 615-0084 (JP)
(72) Inventor: TOKITA, Muneo, OMRON HEALTHCARE CO., LTD., Kyoto-shi, Kyoto 615-0084 (JP)
(74) Representative: Wilhelms . Kilian & Partner Patentanwälte
(86) International application number: PCT/JP2006/311288
(87) International publication number: WO 2006/132221

(57) **Abstract**

A biological information measuring sensor (10) has light-receiving regions (21, 22) arranged on a light-receiving element (20) for receiving light from a living body, and a waveguide (15) including an inlet-side opening portion (14a) into which the light enters and an outlet-side opening portion (14b) from which the light exits, provided corresponding to the light-receiving regions (21, 22), and guiding the light to the light-receiving regions (21, 22). The waveguide (15) has a front-side waveguide (15a) and a rear-side waveguide (15b). The front-side waveguide (15a) is positioned closer to the inlet-side opening portion (14a) and formed such that its opening area gradually increases as it is closer from the inlet-side opening portion (14a) to the outlet-side opening portion (14b), and the rear-side waveguide (15b) is positioned closer to the outlet-side opening portion (14b) and formed such that its opening area gradually reduces as it is closer from the inlet-side opening portion (14a) to the outlet-side opening portion (14b). The construction above enables the biological information measuring sensor to efficiently receive light.

## Description

### TECHNICAL FIELD

The present invention relates to a biological information measuring sensor for measuring biological information in a noninvasive manner by receiving light from a living body.

### BACKGROUND ART

Measurement of biological information is frequently carried out in the medical field. To measure biological information is very important to know the health conditions of a subject. Here, biological information includes a concentration of a particular component included in a living tissue, temperature information, heart rate, blood pressure, and the like. Particular components included in a living tissue to be measured include, for example, glucose, hemoglobin, oxyhemoglobin, neutral fat, cholesterol, albumin, uric acid, and the like included in the blood.

For example, in an ear thermometer, temperature information is measured by receiving radiation light radiated from the eardrum positioned in the auditory meatus. Furthermore, in a glucometer, a technique has been developed to measure a blood glucose concentration spectroscopically in a noninvasive manner by receiving radiation light radiated from the eardrum positioned in the auditory meatus by separating light of a wavelength in a particular range, and then detecting the spectrum of the received light. In addition, in an apparatus for measuring oxygen saturation, a finger sphygmomanometer, and the like, the technique of measuring biological information optically or spectroscopically in a noninvasive manner by receiving light from a living body has been established.

As described above, in the noninvasive measurement method in which light from a living body is received for optical or spectroscopic measurement in a noninvasive manner, it is not necessary to take a living tissue as represented by blood or humor as a sample from a subject, so that the burden on the subject is greatly reduced and the method is suitable as a method of measuring biological information.

When biological information is measured in a noninvasive manner using the optical or spectroscopic measurement method as described above, a biological information measuring sensor is used which receives radiation light emitted from a living body or transmitted light transmitted through a living body or reflected light reflected on a living body by light-receiving means and photoelectrically converting the same for output. The documents disclosing such a biological information measuring sensor include, for example, Japanese Patent Laying-Open No. 2003-70751 (Patent Document 1), Japanese National Publication No. 2001-503999 (Patent Document 2), and the like.
Patent Document 1: Japanese Patent Laying-Open No. 2003-70751
Patent Document 2: Japanese National Publication No. 2001-503999

### DISCLOSURE OF THE INVENTION

### PROBLEMS TO BE SOLVED BY THE INVENTION

In order to measure biological information more accurately in the aforementioned biological information measuring sensor, light-receiving efficiency by light-receiving means should be improved. In particular, when biological information is measured by only capturing radiation light emitted from a living body without provision of a separate light source, improvement of light-receiving efficiency is essential, since radiation light radiating from a living body is very weak.

The present invention is therefore made to solve the aforementioned problem. An object of the present invention is to provide a biological information measuring sensor capable of receiving light from a living body efficiently thereby enabling biological information to be measured with high accuracy.

### MEANS FOR SOLVING THE PROBLEMS

A biological information measuring sensor based on the present invention for measuring biological information in a noninvasive manner by receiving light from a living body includes a light-receiving region and a waveguide. The aforementioned light-receiving region is provided in light-receiving means for receiving light from a living body. The aforementioned waveguide includes an inlet-side opening portion where the light enters and an outlet-side opening portion where the light exits and is provided corresponding to the light-receiving region to introduce the light to the light-receiving region. The aforementioned waveguide includes a first region positioned closer to the inlet-side opening portion and formed to have its opening area gradually increasing from the inlet-side opening portion side toward the outlet-side opening portion side and a second region positioned closer to the outlet-side opening portion and formed to have its opening area gradually reducing from the inlet-side opening portion side toward the outlet-side opening portion side.

Here, "light from a living body" as mentioned above includes radiation light emitted from a living body as well as transmitted light and reflected light of light applied from a light source to a living body.

Because of such a configuration, in the first region of the waveguide, light incident obliquely to the opening surface of the inlet-side opening portion is reflected on the wall surface thereof and thus converted into light traveling in the direction more parallel to the extending direction of the waveguide, while in the second region of the waveguide, its opening area is formed to be gradually narrowed, so that the quantity of light per unit area of light passing through the second region can be increased along its shape. Therefore, the quantity of light incident more vertically to the light-receiving surface can be increased, so that improvement of light-receiving efficiency in the light-receiving region is achieved and biological information can be measured accurately.

In addition, since the opening area is formed to be gradually reduced in the second region of the waveguide, light entering the second region of the waveguide can be condensed at the outlet-side opening portion with the required minimum number of times of reflection on the wall surface of the waveguide. Thus, absorption or scattering of light at the time of reflection is prevented, thereby realizing higher light-receiving efficiency.

Moreover, since the shape of the waveguide closer to the inlet-side opening portion can be tapered, the shape of the tip end of the member forming the waveguide can be tapered accordingly. Therefore, the tip end of the member forming the waveguide can be brought closer to a part to be detected without coming into contact with the surrounding obstruction, and as a result, the inlet-side opening portion can be brought into close proximity to the part to be detected. Therefore, light from a part to be detected can be introduced into the waveguide efficiently. As a result, the light-receiving efficiency in the light-receiving region is improved and biological information can be measured accurately.

In the biological information measuring sensor based on the present invention as described above, preferably, the aforementioned waveguide is formed of an inner circumferential surface of a tubular waveguide formation member. In this case, preferably, an angle between the inner circumferential surface in the first region of the waveguide and a center axis of the waveguide formation member is larger than an angle between the inner circumferential surface in the second region of the waveguide and the center axis of the waveguide formation member.

Here, the angle between the inner circumferential surface of the waveguide formation member and the center axis of the waveguide formation member refers to the narrower angle of the angles between the inner circumferential surface and the center axis.

In order to convert more light into the state closer to a parallel beam in the first region of the waveguide, the inner circumferential surface of the waveguide formation member forming the waveguide needs to be steeper with respect to the center axis. In addition, in order to condense light in the second region of the waveguide without degrading parallelism of light converted into the state closer to a parallel beam, the inner circumferential surface of the waveguide formation member forming the waveguide needs to be gentle. Therefore, by employing the configuration as described above as an example that satisfies these conditions, significant improvement of light-receiving efficiency can be expected.

In the biological information measuring sensor based on the present invention as described above, preferably, the aforementioned waveguide is formed such that an opening shape in the inlet-side opening portion and an opening shape in the outlet-side opening portion are different from each other.

Because of such a configuration, at the inlet-side of the waveguide, the shape of the inlet-side opening portion can be selected so that light enters the waveguide to a maximum extent, while at the outlet-side of the waveguide, the shape of the outlet-side opening portion can be selected according to the shape of the light-receiving region so that the condensed light enters the light-receiving region without loss. Therefore, the higher light-receiving efficiency can be realized.

### EFFECTS OF THE INVENTION

In accordance with the present invention, a biological information measuring sensor capable of receiving light from a living body efficiently can be provided, so that biological information can be measured more accurately.

### BRIEF DESCRIPTION OF THE DRAWINGS

Fig. 1 is a view schematically showing a usage state of a biological information measuring sensor in an embodiment of the present invention.
Fig. 2 is a schematic cross-sectional view of the biological information measuring sensor shown in Fig. 1.
Fig. 3A is a view showing an optical path of light introduced into the biological information measuring sensor shown in Fig. 1 and Fig. 2.
Fig. 3B is a view showing an optical path of light introduced into a biological information measuring sensor in accordance with a conventional example.
Fig. 4 is a schematic diagram illustrating the difference of the angle of incidence of incident light with respect to a light-receiving region in the biological information measuring sensors shown in Fig. 3A and Fig. 3B.
Fig. 5 is a view showing an inclination state of an inner circumferential surface of a waveguide formation member in the present embodiment.
Fig. 6 is a schematic cross-sectional view showing a modification of the biological information measuring sensor in the present embodiment.
Fig. 7 is a view showing a modification of the waveguide formation member of the biological information measuring sensor in the present embodiment.
Fig. 8 is a view showing another modification of the waveguide formation member of the biological information measuring sensor in the present embodiment.
Fig. 9 is a view showing a further modification of the waveguide formation member of the biological information measuring sensor in the present embodiment, in which (a) is a side view of the waveguide formation member, (b) is a view showing the shape of an inlet-side end face of the waveguide formation member, and (c) is a view showing the shape of an outlet-side end face of the waveguide formation member.

### DESCRIPTION OF THE REFERENCE SIGNS

10 biological information measuring sensor, 11 probe portion, 12 protective casing, 13 dustproof film, 13a dustproof window, 14 waveguide formation member, 14A, 14B member, 14a inlet-side opening portion, 14b outlet-side opening portion, 14c inner circumferential surface, 14c1 inlet-side inner circumferential surface, 14c2 outlet-side inner circumferential surface, 15 waveguide, 15a front-side waveguide, 15b rear-side waveguide, 20 light-receiving element, 21, 22 light-receiving region, 23, 24 filter, 30 eardrum (part to be detected), 40a, 40b incident light, A1 first region, A2 second region.

### BEST MODES FOR CARRYING OUT THE INVENTION

In the following, an embodiment of the present invention will be described in detail with reference to the figures. It is noted that in the embodiment illustrated below, a description will be made by way of illustration to a case where the present invention is applied to a glucometer for measuring a blood glucose concentration by putting a probe portion containing a biological information measuring sensor into the auditory meatus, and detecting the spectra of wavelengths in two ranges of mid-infrared radiation radiated from the eardrum using the biological information measuring sensor.

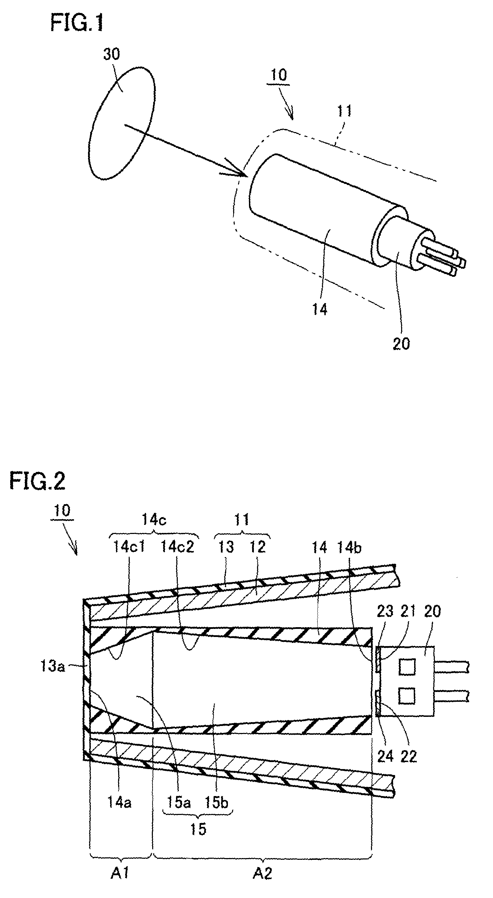

Fig. 1 is a view schematically showing a usage state of a biological information measuring sensor in an embodiment of the present invention, and Fig. 2 is a schematic cross-sectional view of the biological information measuring sensor shown in Fig. 1. In the following, referring to these figures, the structure of the biological information measuring sensor in the present embodiment will be described.

As shown in Fig. 1, a biological information measuring sensor 10 in the present embodiment is included in a detection end of a glucometer to detect weak radiation light radiated from an eardrum 30 as a part to be detected, which is positioned in the auditory meatus. In measurement, as shown in Fig. 1, a probe portion 11 which is the detection end is inserted in the auditory meatus, and the front face (tip end face) of the inserted probe portion 11 is opposed to eardrum 30. In this state, radiation light radiated from eardrum 30 is introduced from the front face of probe portion 11 into probe portion 11, whereby measurement of a blood glucose concentration is performed.

As shown in Fig. 1 and Fig. 2, biological information measuring sensor 10 is mainly configured with a tubular waveguide formation member 14 arranged in the interior of probe portion 11 formed of a protective casing 12 and a dustproof film 13 and a light-receiving element 20 as light-receiving means.

The above-noted protective casing 12 is formed of a tubular member having a front opening. Dustproof film 13 is attached to protective casing 12 to close the front opening of protective casing 12, and in particular, the part closing the front opening of protective casing 12 functions as a dustproof window 13 a. Dustproof film 13 is a film for preventing intrusion of dust into the interior of probe portion 11, and a thin film of, for example, plastic, glass, silicon, or germanium is used for this dustproof film 13. In the present embodiment, a polyethylene film is used so that radiation light radiated from eardrum 30 is transmitted well.

As shown in Fig. 2, waveguide formation member 14 has a waveguide 15 in the interior thereof, and waveguide 15 is defined by an inner circumferential surface 14c of waveguide formation member 14. Waveguide formation member 14 is formed, for example, of a resin material, a metal material or the like, and inner circumferential surface 14c defining waveguide 15 formed in the interior thereof is mirror-finished. A variety of methods can be adopted as the method of mirror finish, and, for example, gold plating and deposition of gold, aluminum or the like are suitable.

Waveguide formation member 14 is arranged such that the front face thereof faces dustproof window 13a. At the back of waveguide formation member 14, the aforementioned light-receiving element 20 is arranged. Waveguide 15 is provided with an inlet-side opening portion 14a on the front face where radiation light radiated from eardrum 30 enters and an outlet-side opening portion 14b on the back face where the aforementioned radiation light passing through waveguide 15 exits.

Waveguide 15 includes a front-side waveguide 15a positioned closer to inlet-side opening portion 14a and a rear-side waveguide 15b positioned closer to outlet-side opening portion 14b. Here, front-side waveguide 15a is a part corresponding to a first region A1 of waveguide 15 and rear-side waveguide 15b is a part corresponding to a second region A2 of waveguide 15. In first region A1 of waveguide 15, an inlet-side inner circumferential surface 14c1 of waveguide formation member 14 is formed in an inclined manner such that the opening area of waveguide 15 gradually increases from inlet-side opening portion 14a toward the outlet-side opening portion 14b side. On the other hand, in second region A2 of waveguide 15, an outlet-side inner circumferential surface 14c2 of waveguide formation member 14 is formed in an inclined manner such that the opening area of waveguide 15 gradually decreases from the inlet-side opening portion 14a side toward outlet-side opening portion 14b.

Light-receiving element 20 arranged at the back of waveguide formation member 14 is an element photoelectrically converting an optical signal received at the light-receiving region as described later into an electrical signal. On the main surface of light-receiving element 20, two light-receiving regions 21, 22 are provided. These light-receiving regions 21, 22 are regions receiving light from a living body. As light-receiving element 20, for example, an element formed of two photodiodes on a single semiconductor substrate may be used or two elements each formed of one photodiode on a single semiconductor substrate may be used.

On the surfaces of light-receiving regions 21, 22, filters 23, 24 are respectively affixed. Filters 23, 24 are spectroscopic means for transmitting only light having a wavelength in a particular range and preventing transmittance of light having a wavelength in the other ranges. In the present embodiment, used as filter 23 is a filter transmitting the mid-infrared radiation with wavelengths of 9 µm-10 µm dependent on a blood glucose concentration, and used as filter 24 is a filter transmitting the mid-infrared radiation with wavelengths of 8 µm-9 µm independent of a blood glucose concentration. Outlet-side opening portion 14b of waveguide 15 formed in the interior of waveguide formation member 14 faces light-receiving regions 21, 22 of light-receiving element 20 with these filters 23, 24 interposed. It is noted that although, in the present embodiment, a filter is used as spectroscopic means by way of illustration, a diffraction grating, a prism, or the like may be used otherwise.

Because of the configuration as described above, radiation light radiated from the eardrum and entering waveguide 15 is condensed inside waveguide 15 to be applied to filters 23, 24. The radiation light applied to filters 23, 24 is separated into mid-infrared radiations of wavelengths in two ranges at filters 23, 24, and then only the separated mid-infrared radiations of wavelengths in the respective ranges enter the respective light-receiving regions 21, 22 of light-receiving element 20. Then, the light received at light-receiving element 20 is photoelectrically converted and output, so that the spectrum is detected based on this output signal in the glucometer main body and the blood glucose concentration is determined.

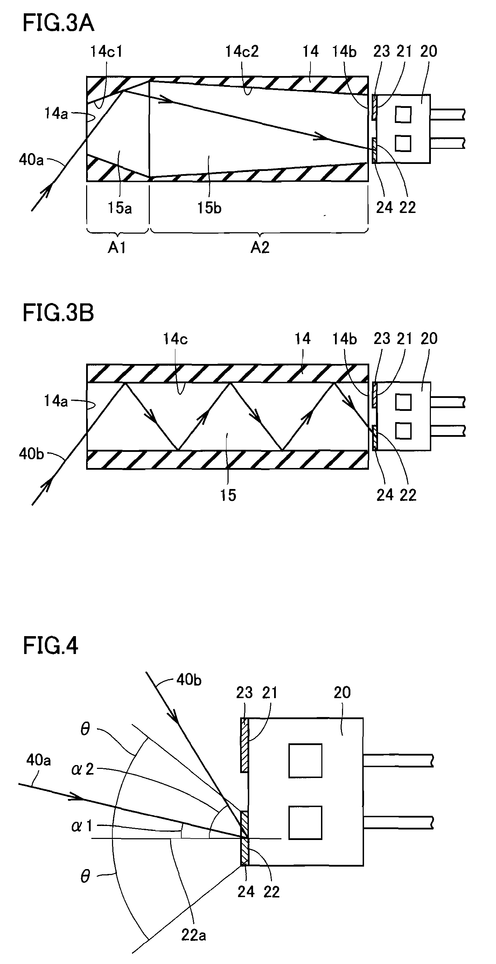

Fig. 3A is a view showing an optical path of light introduced into the biological information measuring sensor shown in Fig. 1 and Fig. 2, and Fig. 3B is a view showing an optical path of light introduced into a biological information measuring sensor according to a conventional example for comparison. Furthermore, Fig. 4 is a schematic diagram illustrating the difference of the angle of incidence of incident light with respect to the light-receiving region in the biological information measuring sensors shown in Fig. 3A and Fig. 3B. In the following, referring to these figures, improvement of light-receiving efficiency which is an effect achieved by the biological information measuring sensor in the present embodiment will be described.

As shown in Fig. 3A, in biological information measuring sensor 10 in the present embodiment, radiation light 40a incident from a diagonal direction at a prescribed angle to the opening surface of inlet-side opening portion 14a of waveguide formation member 14 impinges on inlet-side inner circumferential surface 14c1 of waveguide formation member 14 defining front-side waveguide 15a and is reflected on this inlet-side inner circumferential surface 14cl. As described above, since inlet-side inner circumferential surface 14c1 of waveguide formation member 14 has a prescribed inclined shape, radiation light 40a reflected on inlet-side inner circumferential surface 14cl is converted into light traveling in a direction more parallel to the extending direction of waveguide 15 (that is, the direction in which the center axis of waveguide formation member 14 extends). Therefore, because of the presence of this front-side waveguide 15a, the radiation light introduced into rear-side waveguide 15b is adjusted to a state closer to a parallel beam.

Then, the radiation light entering rear-side waveguide 15b is condensed along the shape of outlet-side inner circumferential surface 14c2 of waveguide formation member 14 defining rear-side waveguide 15b and enters light-receiving regions 21, 22 of light-receiving element 20 through outlet-side opening portion 14b. Here, rear-side waveguide 15b is formed such that its opening area is gradually reduced from the inlet-side opening portion 14a side toward outlet-side opening portion 14b, so that the radiation light entering rear-side waveguide 15b is reflected on outlet-side inner circumferential surface 14c2 with the required minimum number of times. Therefore, absorption or scattering of radiation light at the time of reflection is prevented and reduction of the quantity of light exiting from outlet-side opening portion 14b is prevented.

On the other hand, as shown in Fig. 3B, in the biological information measuring sensor according to a conventional example with waveguide formation member 14 shaped like a straight pipe, radiation light 40b incident from a diagonal direction at a prescribed angle to the opening surface of inlet-side opening portion 14a of waveguide formation member 14 is repeatedly reflected by inner circumferential surface 14c of waveguide formation member 14 defining waveguide 15a, passes through outlet-side opening portion 14b without being converted into light traveling in the direction more parallel to the extending direction of waveguide 15, and reaches light-receiving regions 21, 22 of light-receiving element 20 as it is. Due to the repeated reflection on inner circumferential surface 14c, radiation light 40b is partially absorbed or scattered by inner circumferential surface 14c and thus enters light-receiving regions 21, 22 with the reduced quantity of light.

Here, as shown in Fig. 4, light-receiving element 20 has an angle range in which light incident on light-receiving regions 21, 22 can be received, and this is generally called angular aperture. In Fig. 4, this angular aperture is represented by angle θ. This angular aperture is an angle indicating the inclination from the normal to the light-receiving surfaces of light-receiving regions 21, 22, and is the critical angle at which the incident light cannot be received when the angle between the incident light and the normal is equal to or greater than a prescribed angle (that is, when the incident light is incident on the receiving surface at an angle greater than the angular aperture).

As described above, in biological information measuring sensor 10 in the present embodiment, radiation light 40a incident obliquely to the opening surface of inlet-side opening portion 14a of waveguide formation member 14 is also reflected on inlet-side inner circumferential surface 14c1 defining front-side waveguide 15a and thus converted into light traveling in the direction more parallel to the extending direction of waveguide 15. Therefore, the converted radiation light enters light-receiving regions 21, 22 in such a state in which it approaches more parallel to the normal to the light-receiving surfaces of light-receiving regions 21, 22. By contrast, in the biological information measuring sensor according to the conventional example, radiation light 40b incident obliquely to the opening surface of inlet-side opening portion 14a of waveguide formation member 14 enters light-receiving regions 21, 22 with the same angle kept with respect to the normal to the light-receiving surfaces of light-receiving regions 21, 22.

Therefore, as shown in Fig. 3A and Fig. 3B, radiation lights 40a, 40b incident obliquely at the same angle to the opening surface of inlet-side opening portion 14a of waveguide formation member 14 enter at different angles to the light-receiving surfaces of light-receiving regions 21, 22, as shown in Fig. 4. If the angles between radiation lights 40a, 40b and the normal to the light-receiving surface of light-receiving regions 21, 22 are respectively α1, α2, the relation between these angles and the angular aperture may be α1< θ < α2 in some instances. Therefore, radiation light that is not received by the biological information measuring sensor according to the conventional example may be received by biological information measuring sensor 10 in the present embodiment, and it can be understood that biological information measuring sensor 10 in the present embodiment is greatly improved in terms of light-receiving efficiency as compared with the biological information measuring sensor according to the conventional example.

As explained above, with biological information measuring sensor 10 in the present embodiment, in front-side waveguide 15a, radiation light incident obliquely to the opening surface of inlet-side opening portion 14a is reflected on inlet-side inner circumferential surface 14c 1 and thus converted into light traveling in the direction more parallel to the extending direction of waveguide 15, while in rear-side waveguide 15b, its opening area is formed to be gradually narrowed, so that the quantity of light per unit area of radiation light passing through rear-side waveguide 15b can be increased along the shape. Therefore, the quantity of light incident more vertically to light-receiving regions 21, 22 can be increased, so that improvement of light-receiving efficiency in light-receiving regions 21, 22 is achieved and biological information can be measured accurately.

In addition, since the opening area is formed to be gradually reduced in rear-side waveguide 15b, radiation light entering rear-side waveguide 15b can be condensed at outlet-side opening portion 14b with the required minimum number of times of reflection on outlet-side inner circumferential surface 14c2 of rear-side waveguide 15b. Thus, absorption or scattering of light at the time of reflection is prevented, thereby realizing higher light-receiving efficiency.

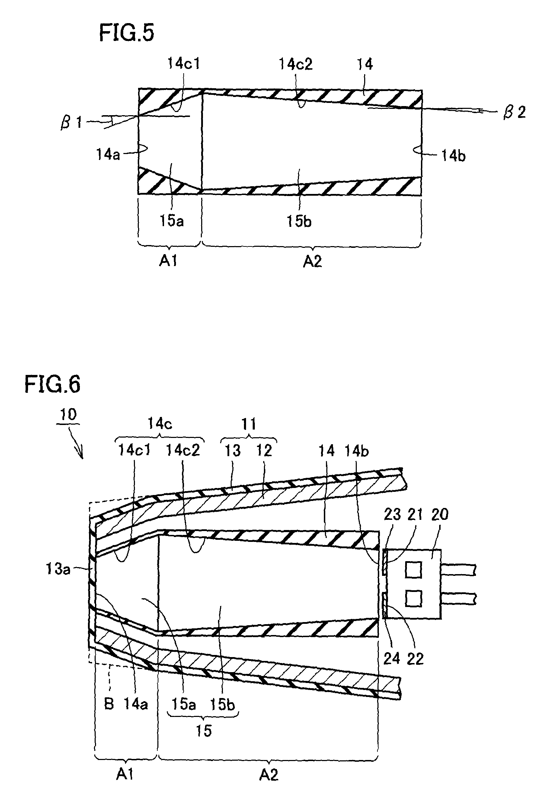

Fig. 5 is a view showing the inclination state of the inner circumferential surface of the waveguide formation member in the present embodiment. In order to convert more radiation light into the state closer to a parallel beam in front-side waveguide 15a, inlet-side inner circumferential surface 14c1 defining front-side waveguide 15a needs to be steeper with respect to the center axis of waveguide formation member 14. In addition, in order to condense radiation light in rear-side waveguide 15b without degrading parallelism of light converted into the state closer to a parallel beam in front-side waveguide 15a, outlet-side inner circumferential surface 14c2 defining rear-side waveguide 15b needs to be gentle with respect to the center axis of waveguide formation member 14.

As one of conditions that satisfy this, it is conceived that the angle between inlet-side inner circumferential surface 14c1 defining front-side waveguide 15a and the center axis of waveguide formation member 14 is formed to be larger than the angle between outlet-side inner circumferential surface 14c2 defining rear-side waveguide 15b and the center axis of waveguide formation member 14. Here, the angles between inlet-side and outlet-side inner circumferential surfaces 14cl, 14c2 and the center axis of waveguide formation member 14 refer to the narrower angles, of the angles between these inlet-side and outlet-side inner circumferential surfaces 14c1, 14c2 and the center axis of waveguide formation member 14.

By satisfying such a condition, more radiation light can be converted into the state closer to a parallel beam in front-side waveguide 15a while the radiation light can be condensed in rear-side waveguide 15b without degrading the parallelism of the light converted into the state closer to a parallel beam in front-side waveguide 15a, so that significant improvement of light-receiving efficiency can be expected.

Fig. 6 is a schematic cross-sectional view showing a modification of the biological information measuring sensor in the present embodiment. In the biological information measuring sensor in accordance with the present modification, the shape of the tip end of the waveguide formation member is tapered according to the shape of front-side waveguide 15a. In addition, the tip end shape of probe portion 11 of the glucometer is tapered according to this waveguide formation member 14 having a tapered shape.

Because of such a configuration, as compared with the glucometer including the biological information measuring sensor shown in Fig. 2, the tip end shape of probe portion 11 can be narrowed by the amount shown by broken line B in Fig. 6, so that when probe portion 11 is inserted into the auditory meatus, the tip end face of probe portion 11 can be inserted into a deeper portion of the auditory meatus. Therefore, inlet-side opening portion 14a of waveguide formation member 14 can be brought closer to eardrum 30 as a part to be detected. As a result, radiation light emitted from eardrum 30 can be introduced into waveguide 15 efficiently. Therefore, the light-receiving efficiency in light-receiving regions 21, 22 is improved and biological information can be measured accurately.

In the foregoing description, the description has been made by way of illustration to the case where the present invention is applied to the biological information measuring sensor incorporated in a glucometer measuring a blood glucose concentration by detecting the spectra of wavelengths in two ranges of mid-infrared radiation. However, the present invention is also applicable to a biological information measuring sensor incorporated in a measuring apparatus detecting any other biological component or a biological information measuring sensor incorporated in a measuring apparatus measuring temperature information, pulse rate, blood pressure, or the like. In the former case, the present invention is additionally applicable to, for example, one using mid-infrared radiation as well as one using near-infrared radiation or one using visible light. Furthermore, the components to be detected include, in addition to glucose included in the blood as described above, hemoglobin, oxyhemoglobin, neutral fat, cholesterol, albumin, uric acid, and the like.

When objects to be measured are varied, the configuration of the biological information measuring sensor needs to be modified in various manners. When the object to be measured is glucose as described above, the configuration of the biological information measuring sensor is also susceptible to a variety of modifications. In the following, examples of them will be described.

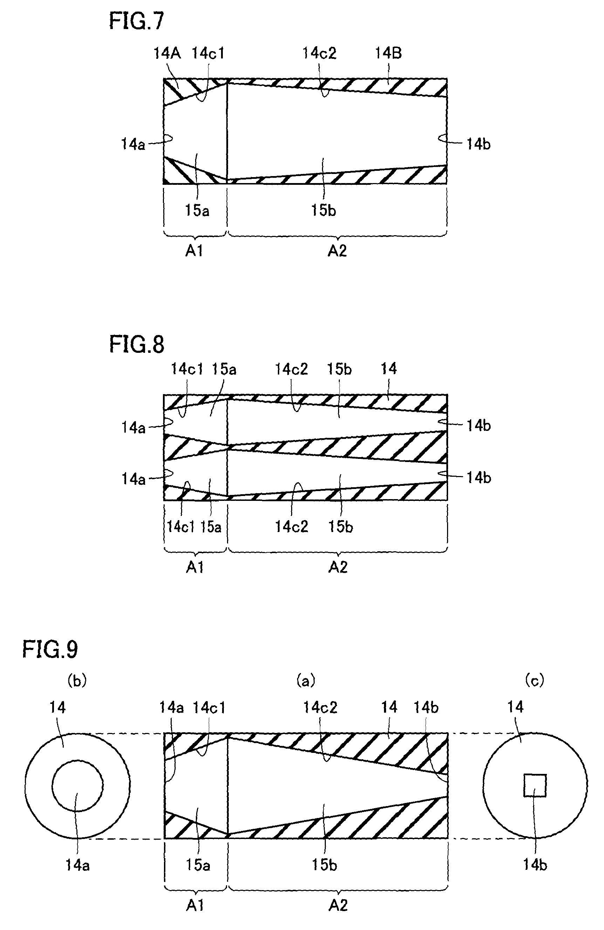

Fig. 7 to Fig. 9 are views of modifications of the waveguide formation member of the biological information measuring sensor in the present embodiment. It is noted that, in Fig. 9, (a) is a side view of the waveguide formation member, (b) is a view showing the shape of the inlet-side end face of the waveguide formation member, and (c) is a view showing the shape of the outlet-side end face of the waveguide formation member. In the following, referring to these figures, modifications of the waveguide formation member of the biological information measuring sensor in the present embodiment will be described.

A modification shown in Fig. 7 is formed by dividing waveguide formation member 14 into two members 14A, 14B in the direction in which the center axis extends and combining these members 14A, 14B by adhesion or the like for integration. Front-side waveguide 15a is provided in member 14A and rear-side waveguide 15b is provided in member 14B.

In this manner, in the case where the waveguide formation member is formed by combining and integrating a plurality of members each having a waveguide formed therein with each other, the effect similar to the effect in the present embodiment as described above is also achieved. The advantage of combining and integrating a plurality of members with each other in this manner is, for example, as follows. When the waveguide formation member is fabricated by cutting a metal or molding a resin, shaping is difficult by such processing, and the employment of this configuration makes fabrication easier even in such a case.

In a modification shown in Fig. 8, two waveguides 15 are formed in waveguide formation member 14. These two waveguides 15 are provided corresponding to two light-receiving regions provided at a not-shown light-receiving element, and the respective radiation light entering waveguides 15 from inlet-side opening portions 14a provided on the front face of waveguide formation member 14 passes through the respective waveguides 15, is condensed and exits at the respective outlet-side opening portions 14b provided on the back face of waveguide formation member 14, and is then respectively applied to the two light-receiving regions of the not-shown light-receiving element.

Because of such a configuration, the respective shapes of outlet-side opening portions 14b of waveguide formation member 14 can be respectively adopted to the shapes of the light-receiving regions, so that the radiation light introduced into waveguides 15 can be photoelectrically converted by the light-receiving element without loss. Therefore, biological information can be measured with higher accuracy.

In a modification shown in Fig. 9, the opening shape of inlet-side opening portion 14a of waveguide formation member 14 and the opening shape of outlet-side opening portion 14b of waveguide formation member 14 are different from each other. Specifically, the opening shape of inlet-side opening portion 14a is circular and the opening shape of outlet-side opening portions 14b is rectangular.

In this manner, in the case where the inlet-side opening portion and the outlet-side opening portion of the waveguide have different opening shapes, advantageously, at the inlet side of the waveguide, the shape of the inlet-side opening portion can be selected so that light enters the waveguide to a maximum extent, while at the outlet side of the waveguide, the shape of the outlet-side opening portion can be selected according to the shape of the light-receiving region so that the condensed light enters the light-receiving region without loss. Therefore, the higher light-receiving efficiency can be realized.

In the embodiment as described above, the description has been made by way of illustration to the biological information measuring sensor receiving radiation light radiated from a part to be measured of a living body. However, the present invention is also applicable to a biological information measuring sensor receiving transmitted light or reflected light of light applied from a light source to a part to be measured of a living body and photoelectrically converting the same, as a matter of course.

Furthermore, in the embodiment and modifications thereof as described above, the description has been made by way of illustration assuming that a part to be measured of a living body is an eardrum. However, a part to be measured is not limited thereto and may be various parts of a living body.

In this manner, the foregoing embodiment and modifications thereof as disclosed herein are illustrative rather than limitative in all respects. The technical scope of the present invention is defined by the claims, and it is intended that equivalents to the claims and all modifications within the scope are embraced.

## Claims

1. A biological information measuring sensor for measuring biological information in a noninvasive manner by receiving light from a living body, comprising:
a light-receiving region provided in light-receiving means for receiving light from a living body; and
a waveguide including an inlet-side opening portion where said light enters and an outlet-side opening portion where said light exits and being provided corresponding to said light-receiving region to introduce said light to said light-receiving region, wherein
said waveguide includes a first region positioned closer to said inlet-side opening portion and formed to have its opening area gradually increasing from said inlet-side opening portion side toward said outlet-side opening portion side and a second region positioned closer to said outlet-side opening portion and formed to have its opening area gradually reducing from said inlet-side opening portion side toward said outlet-side opening portion side.

2. The biological information measuring sensor according to claim 1, wherein
said waveguide is formed of an inner circumferential surface of a tubular waveguide formation member, and
an angle between the inner circumferential surface in said first region of said waveguide and a center axis of said waveguide formation member is larger than an angle between the inner circumferential surface in said second region of said waveguide and the center axis of said waveguide formation member.

3. The biological information measuring sensor according to claim 1, wherein in said waveguide, an opening shape in said inlet-side opening portion and an opening shape in said outlet-side opening portion are different from each other.
